**Europäisches Patentamt**

**European Patent Office**

**Office européen des brevets**

(19)

(11) Veröffentlichungsnummer : **0 111 203 B2**

(12) # NEUE EUROPÄISCHE PATENTSCHRIFT

(45) Veröffentlichungstag der neuen Patentschrift :
05.06.91 Patentblatt 91/23

(51) Int. Cl.$^5$ : **C07C 19/045**, C07C 17/02

(21) Anmeldenummer : 83111651.2

(22) Anmeldetag : 22.11.83

(54) **Verfahren zur Herstellung von 1,2-Dichlorethan.**

Die Akte enthält technische Angaben, die nach dem Eingang der Anmeldung eingereicht wurden und die nicht in dieser Patentschrift enthalten sind.

(30) Priorität : 08.12.82 DE 3245366

(43) Veröffentlichungstag der Anmeldung :
20.06.84 Patentblatt 84/25

(45) Bekanntmachung des Hinweises auf die Patenterteilung :
14.01.87 Patentblatt 87/03

(45) Bekanntmachung des Hinweises auf die Entscheidung über den Einspruch :
05.06.91 Patentblatt 91/23

(84) Benannte Vertragsstaaten :
BE CH DE FR GB IT LI NL SE

(56) Entgegenhaltungen :
EP-A- 0 058 976
EP-A- 0 082 342
DE-A- 1 568 583
DE-A- 3 202 378
NL-A- 6 901 398
Ullmann, Band 9, Seiten 426–428
Ullmans Encyklopädie der technischen Chemie, 4. Auflage, 9, 1976, Seite 363

(73) Patentinhaber : HOECHST AKTIENGESELLSCHAFT
Postfach 80 03 20
W-6230 Frankfurt am Main 80 (DE)

(72) Erfinder : Hundeck, Joachim, Dr.
Argelander Strasse 135
W-5300 Bonn (DE)
Erfinder : Scholz, Harald, Dr.
Am Beissel 8
W-5042 Erftstadt (DE)
Erfinder : Hennen, Hans
Kendenicher Strasse 86
W-5030 Hürth (DE)

EP 0 111 203 B2

## Beschreibung

Die Herstellung von 1,2-Dichlorethan durch Reaktion von Ethylen mit Chlor in 1,2-Dichlorethan als Lösungsmittel und Reaktionsmedium ist bekannt. Als hauptsächliches Nebenprodukt fällt bei dieser Reaktion 1,1,2-Trichlorethan durch Substitution von Dichlorethan an. Zur Unterdrückung dieser Substitutionsreaktion verwendet man als Katalysatoren neben den Chloriden der Elemente der IV. bis VI. Gruppe des Periodensystems vor allem wasserfreies Eisen-III-chlorid, zum Teil bei gleichzeitiger Gegenwart von Sauerstoff, da Eisen-III-chlorid leicht zugänglich und preisgünstig ist.

Das gebildete rohe, katalysatorhaltige Dichlorethan wird im allgemeinen aus dem Reaktionsgefäß abgezogen und zur Entfernung des Katalysators und des im Rohprodukt enthaltenen Chlorwasserstoffs mit Wasser bzw. wäßrigen Alkalilösungen behandelt und anschließend nach bekannten Verfahren destillativ aufgearbeitet.

Die Verwendung von FeCl$_3$ als Kasalysator bei der Additionschlorierung von Ethylen ist mit gewissen Nachteilen verbunden. So wirkt FeCl$_3$ in Gegenwart von Wasser korrosiv gegenüberden metallischen Werkstoffen von Reaktoren, Kolonnen oder Wärmeaustauschern, soweit diese mit dem Katalysator in Berührung kommen. Das zur Chlorierung normalerweise verwendete Chlor mit technischem Reinheitsgrad enthält stets Spuren von Feuchtigkeit ; außerdem entsteht immer Chlorwasserstoff aus unerwünschten Nebenreaktionen.

Sofern die bei der Chlorierung von Ethylen freigesetzte Wärmeenergie nutzbar gemacht werden soll muß die Reaktion bei Temperaturen oberhalb des Siedepunktes von Dichlorethan bei Normaldruck durchgeführt werden. Da mit steigender Temperatur die Korrosionsrate erheblich zunimmt, ist es unumgänglich, die Apparaturen für die Chlorierungsreaktion mit korrosionsfesten Werkstoffen auszustatten, wodurch die Wirtschaftlichkeit des angewandten Verfahrens beeinträchtigt wird.

Die EP-A-58 976 betrifft ein Verfahren zur Herstellung von 1,2-Dichlorethan in der Gasphase in Gegenwart eines kupfer- und gegebenenfalls eisensalzhaltigen Katalysators, der zwecks Herabsetzung der Flüchtigkeit des Kupfer-II-Chlorids zusätzlich Alkalimetall- oder Erdalkalimetallchloride enthalten kann. Aus Ullmann's Enzyklopädie der Technischen Chemie, Band 9 (1975), Seite 427, linke Spalte, 4. Absatz, und Seite 428, linke Spalte, 1. Absatz, geht hervor, daß man zur Chlorierung von Ethylen in flüssigerPhase als Katalysator Eisen-III-Chlorid oder Antimonchloride oder Kupferchlorid einsetzen kann, während für die Chlorierung von Ethylen in der Gasphase als Katalysator Eisen-III-Chlorid auf einem Träger oder Kalziumchlorid oder Bleisalze infrage kommen. Weiterhin beschreibt die DE-A-1 568 583 die Herstellung von Dichlorethanin der Gasphase mit Natriumchlorid und/oder Kaliumchlorid als Katalysator. Schließlich beschreibt die nicht vorveröffentlichte EPA-82 342 ein Katalysatorgemisch zur Herstellung von 1,2-Dichlorethan, welches aus Eisen-III-Chlorid und einer etwa äquivalenten Menge einer Stickstoffbase oder eines Salzes dieser Base besteht. Von einer Bildung von Tetrachloroferraten (1-) im Reaktionsgemisch ist dabei nicht die Rede.

Es wurde nunmehr gefunden, daß die durch FeCl$_3$ als Katalysator bei der Herstellung von 1,2-Dichlorethan verursachte Korrosion in nicht korrosionsfesten Reaktoren erheblich vermindert werden kann, wenn man wasserfreie Tetrachloroferrate als Katalysatoren verwendet. Darüberhinaus wurdefestgestellt, daß diese Verbindungen sich auch vorteilhaft auf die Nebenproduktbildung, welche dadurch verringert wird, auswirken.

Gegenstand der Erfindung ist somit ein Verfahren zur Herstellung von 1,2-Dichlorethan in 1,2-Dichlorethan als Lösungsmittel durch Reaktion von Ethylen mit Chlor in Gegenwart eines Metallhalogenids, welches die Eigenschaften einer Lewis-Säure hat, und eines Halogenids eines Metalles der Gruppe I A und/oder II A des "Periodisches System der Elemente" als Katalysator bei einer Temperatur von etwa 20 bis 200°C sowie Normal- oder Überdruck und Abtrennen des 1,2-Dichlorethans aus dem Chlorierungsgemisch durch Destillation, welches dadurch gekennzeichnet ist, daß man als Katalysator wasserfreies Natrium-tetrachloroferrat (1-) (NaFeCl$_4$), Kalium-tetrachloroferrat (1-) (KFeCl$_4$) oder Magnesium-bis-[tetrachloroferrat (1-)] (Mg[FeCl$_4$]$_2$) verwendet oder solche Substanzen in stöchiometrischen Mengen einsetzt, die im Reaktionsgemisch diese genannten Tetrachloroferrate (1-) bilden und die Chlorierung in Gegenwart von Sauerstoff oder Luft durchführt, wobei die Konzentration des Katalysators etwa 0,005 bis 0,5 Gew%, berechnet als Eisen-III-chlorid und bezogen auf die Menge des Lösungsmittels, beträgt.

Die Herstellung der Katalysatoren kann in bekannter Weise erfolgen.

Der erfindungsgemäße Katalysator wird im allgemeinen in dem im Reaktor vorgelegten Lösungsmittel gelöst oder suspendiert. Das Tetrachloroferrat (1-) kann aber auch außerhalb der Reaktionslösung zubereitet und in den Reaktor eingebracht werden. Es ist ferner möglich, das im Reaktor vorgelegteLösungsmittel mit wasserfreiem FeCl$_3$ und einer im Reaktionsmedium löslichen wasserfreien weiterenKomponente, die das Tetrachloroferrat (1-) zu bilden vermag, zu beschicken. Schließlich kann das Tetra-chloroferrat (1-) im Reaktionsgemisch derart gebildet werden, daß man das Reaktionsgemisch z.B. mit einem Tetrachloroferrat (2-) beschickt und letzteres Anion im Reaktionsmedium zu einem Tetra-chloroferrat(1-) oxidiert.

Die erfindungsgemäßen Katalysatoren sind als technisch fortschrittlich zu bezeichnen, da durch sie die bei bekannten Verfahren zur Herstellung von 1,2-Dichlorethan auftretende und nachteilig sich auswirkende

2

Korrosion bei Verwendung von nicht korrosionsbeständigen, metallischen Reaktoren in einem erheblichen Maße zurückgedrängt wird. Weiterhin wurde festgestellt, daß mit Ausnahme von geringen Mengen des ersten Substitutionsproduktes 1,1,2-Trichlorethan und einer entsprechend geringen Menge Chlorwasserstoff unter den erfindungsgemäßen Verfahrensbedingungen so gut wie keine weiteren Nebenprodukte gebildet werden. Die Reaktionslösung bleibt auch bei längerer Raktionsdauer hell, wenn Tetrachloroferrate (1-) in der Reaktionslösung vorliegen. Ein gegebenenfalls im Zuge der Reaktion bereits dunkel gefärbtes Reaktionsgemisch hellt sich im Ausmaß der Zugabe der genannten Verbindungen beim weiteren Reaktionsverlauf wieder auf. Schließlich ist festzustellen, daß beim Verfahren der Erfindung der Umsatz bei hoher Raum/Zeit-Ausbeute nahezu quantitativ ist.

Das Verfahren der Erfindung kann beispielsweise in dem in der DE-OS 24 27 045 beschriebenen Schlaufenreaktor oder in jedem anderen für die Durchführung des Verfahrens geeigneten Reaktor ausgeführt werden.

Der Gegenstand der Erfindung sei anhand der nachfolgenden Beispiele näher erläutert :

## Beispiele 1 und 2

In einem Schlaufenreaktor aus Glas mit einem Fassungsvermögen von etwa 2 1 wurden 2,0 kg 1,2-Dichlorethan, das 0,1-0,3 Gew% eines in der Tabelle aufgeführten Katalysators gelöst enthält, vorgelegt. Der aufsteigende Teil der Reaktorschlaufe enthielt eine Füllkörperschicht. Unterhalb der Füllkörperschicht befanden sich Einleitungsrohre in den Reaktor für Ethylen, Chlor und Luft, durch die jeweils ca. 60 l/h Chlor und Ethylen sowie 15 l/h Luft eingespeist wurden. Die Reaktorflüssigkeit wurde im Reaktorsystem nach dem Mammutpumpenprinzip umgewälzt. Während der Reaktion stellte sich im Reaktionsgemisch eine Temperatur von etwa 77°C ein.

In einem über dem Reaktor angeordneten Wasserkühler wurden die aus dem Reaktor abziehenden Dichlorethan-Dämpfe kondensiert und anschließend ein der produzierten Menge entsprechender Teil des Kondensates mittels eines Kondensatteilers abgezweigt und entnommen, während überschüssiges Kondensat in die Reaktionszone zurückgeleitet wurde. Mittels einer Kühlfalle wurde ein weiterer Dichlorethan-Anteil aus dem überwiegend aus Inertgasen bestehenden Abgas abgeschieden. Das erhaltene Roh-Dichlorethan besaß die in der Tabelle dargestellte Zusammensetzung. Die Ausbeute an Roh-Dichlorethan betrug durchschnittlich 267 g pro Stunde, wobei zu berücksichtigen ist, daß die eingegebenen Gasmengen lediglich mittels Strömungsmeßgeräten bestimmt worden sind. Trotz eines in den Reaktor eingelegten Stahlkörpers blieb der Eisengehalt im Reaktionsgemisch unverändert.

## Beispiel 3

Es wurde analog Beispiel 1 verfahren, wobei jedoch in den Reaktor 2072 g 1,2-Dichlorethan, in welchem 1,6 g $FeCl_3$ gelöst waren, vorgelegt wurden. Gemäß kolorimetrischer Bestimmung betrug der $FeCl_3$-Gehalt in der Lösung 0,076 Gew%. Bei der Reaktion der in den Reaktor eingeleiteten Mengen an Ethylen und Chlor in Gegenwart von Luft wurde ein Roh-Dichlorethan erhalten mit einem Gehalt von 0,18 Gew% 1,1,2-Trichlorethan.

Der Reaktionslösung wurden danach 0,5 g $MgCl_2$ zugefügt. Während der weiteren Versuchsdauer von 4 Wochen in kontinuierlichem Betrieb wurden stündlich etwa 265 g Rohprodukt erhalten. Der Eisengehalt im Reaktionsgemisch blieb trotz eines in den Reaktor eingelegten Stahlkörpers unverändert.

Die Analyse des im Kondensator anfallenden 1,2-Dichlor-ethans (Produkt A) bzw. der im Reaktor verbliebenen Flüssigkeit (Produkt B) ergab folgende Werte :

EP 0 111 203 B2

|  | Produkt A (Gew%) | Produkt B (Gew%) |
|---|---|---|
| $C_2H_5Cl$ | < 0,002 | < 0,002 |
| 1,2-EDC | 99,97 | 99,84 |
| 1,1,2-ETC | 0,021 | 0,089 |
| HCl | < 0,001 | -- |
| Sonstige | 0,007 | 0,07 |

EDC = 1,2-Dichlorethan

ETC = 1,1,2-Trichlorethan

**Beispiel 4**

Es wurde analog Beispiel 3 verfahren, wobei jedoch in den Reaktor 2155 g 1,2-Dichlorethan, in welchem zuvor 1,7 g Eisen-III-chlorid gelöst worden waren, vorgelegt wurden. Gemäß kolorimetrischer Eisenbestimmung betrug der $FeCl_3$-Gehalt in der Lösung 0,083 Gew%. Bei der Reaktion der in den Reaktor eingeleiteten Mengen an Ethylen und Chlor in Gegenwart von Luft wurde ein Roh-Dichlorethan mit einem Gehalt von 0,12 Gew% 1,1,2-Trichlorethan erhalten.

Der Reaktionslösung wurden danach 0,6 g NaCl zugeführt und weiteres 1,2-Dichlorethan hergestellt. Die Analyse des im Kondensator anfallenden 1,2-Dichlorethans (Produkt A) ergab folgende Werte :

|  | Produkt A (Gew%) |
|---|---|
| $C_2H_5Cl$ | < 0,002 |
| 1,2-EDC | 99,97 |
| 1,1,2-ETC | 0,025 |
| HCl | < 0,001 |
| Sonstige | 0,004 |

Nach einer Versuchsdauer von 10 Tagen in kontinuierlichem Betrieb wurden weitere 1,7 g $FeCl_3$ und 0,6 g NaCl in die Reaktorflüssigkeit zugegeben, so daß der Eisen-III-chlorid-Gehalt in der Lösung 0,164 Gew% betrug. Die Analyse des nunmehr im Kondensator anfallenden Produktes A ergab folgende Werte :

|  | Produkt A (Gew%) |
|---|---|
| $C_2H_5Cl$ | < 0,002 |
| 1,2-EDC | 99,98 |
| 1,1,2-ETC | 0,012 |
| HCl | 0,001 |
| Sonstige | 0,004 |

Der Versuch wurde 7 Tage in kontinuierlichem Betrieb fortgesetzt. Darauf wurden nochmals 1,7 g $FeCl_3$ und 0,6 g NaCl der Reaktorflüssigkeit zugefügt, so daß der Eisen-III-chlorid-Gehalt in der Lösung 0,248 Gew% betrug. Das im Kondensator anfallende Produkt A bzw. die Reaktorflüssigkeit B besaß folgende Zusammensetzung :

4

|  | Produkt A (Gew%) | Produkt B (Gew%) |
|---|---|---|
| $C_2H_5Cl$ | <0,002 | <0,002 |
| 1,2-EDC | 99,97 | 99,81 |
| 1,1,2-ETC | 0,018 | 0,068 |
| HCl | <0,001 | - |
| Sonstige | 0,004 | 0,12 |

**Beispiel 5**

In einem Rührgefäß mit einem Volumen von 5 l wurde ein Gemisch aus 4341 g 1,2-Dichlorethan und 3,3 g FeCl$_3$ vorgelegt. Der kolorimetrisch ermittelte FeCl$_3$-Gehalt betrug 0,076 Gew%. Anschließend wurden der magnetisch gerührten Lösung 1,1 g Na$_2$CO$_3$ zugefügt. In das vorgelegte Gemisch wurde über ein Einleitungsrohr stündlich 60 l Chlorgas und 15 l Luft und über ein weiteres Rohr mit Glasfritte 60 l/h Ethylen eingeleitet.

In einem über dem Reaktor angeordneten Wasserkühler wurden die aus dem Reaktor abziehenden Dichlorethandämpfe kondensiert und anschließend ein der produzierten Menge Dichloretnan entsprechender Teil des Kondensates mittels eines Kondensatteilers abgezweigt und entnommen, während überschüssiges Kondensat in die Reaktionszone zurückgeleitet wurde. Mittels einer Kühlfalle wurde ein weiterer KondensatAnteil aus dem überwiegend aus Inertgasen bestehenden Abgas abgeschieden.

Die Analyse des im Kondensator anfallenden Produktes A ergab folgende Werte :

|  | Produkt A (Gew%) |
|---|---|
| $C_2H_5Cl$ | <0,002 |
| 1,2-EDC | 99,96 |
| 1,1,2-ETC | 0,031 |
| HCl | 0,002 |
| Sonstige | 0,005 |

Nach 60-stündiger Versuchsdauer wurde die Lösung in dem Maße mit 1,2-Dichlorethan verdünnt, daß der kolorimetrisch ermittelte Eisen-III-chlorid-Gehalt noch 0,028 Gew% betrug. Nach einer Laufzeit von weiteren 40 Stunden ergab die Analyse des im Kondensator anfallenden Produktes A folgende Werte :

|  | Produkt A (Gew%) |
|---|---|
| $C_2H_5Cl$ | <0,002 |
| 1,2-EDC | 99,96 |
| 1,1,2-ETC | 0,03 |
| HCl | 0,001 |
| Sonstige | 0,003 |

TABELLE

| Beispiel | Katalysator | A | Analyse Roh-Produkt (Gew%) | | | | |
|---|---|---|---|---|---|---|---|
| | | | $C_2H_5Cl$ | 1,2-EDC | 1,1,2-ETC | HCl | Sonst. |
| 1 | $NaFeCl_4$ | 0,14 | <0,002 | 99,94 | 0,03 | 0,002 | 0,005 |
| 2 | $KFeCl_4$ | 0,24 | <0,002 | 99,86 | 0,13 | 0,003 | 0,006 |

A = Konzentration des Katalysators berechnet als $FeCl_3$

EP 0 111 203 B2

**Ansprüche**

1. Verfahren zur Herstellung von 1,2-Dichlorethan in 1,2-Dichlorethan als Lösungsmittel durch Reaktion von Ethylen mit Chlor in Gegenwart eines Metallhalogenids, welches die Eigenschaften einer Lewis-Säure hat, und eines Halogenids eines Metalles der Gruppe I A und/oder II A des "Periodisches System der Elemente" als Katalysator bei einer Temperatur von etwa 20 bis 200°C sowie Normal-oder Überdruck und Abtrennen des 1,2-Dichlorethans aus dem Chlorierungsgemisch durch Destillation, dadurch gekennzeichnet, daß man als Katalysator wasserfreies Natrium-tetrachloroferrat (1-) (NaFeCl$_4$), Kalium-tetrachloroferrat (1-) (KFeCl$_4$) oder Magnesium-bis-[tetrachloroferrat (1-)] (Mg[FeCl]$_2$ verwendet oder solche Substanzen in stöchiometrischen Mengen einsetzt, die im Reaktionsgemisch diese genannten Tetrachloroferrate (1-) bilden, und die Chlorierung in Gegenwart von Sauerstoff oder Luft durchführt wobei die Konzentration des Katalysators etwa 0,005 bis 0,5 Gew%, berechnet als Eisen-III-chlorid und bezogen auf die Menge des Lösungsmittels, beträgt.

**Claims**

1. Process for making 1,2-dichloroethane in 1,2-dichloroethane as solvent by reacting ethylene with chlorine in the presence of a metallic halide having the properties of a Lewis acid and of a halide of a metal of the group I A and/or II A of the "Periodic Table of Elements" as the catalyst at a temperature of about 20 to 200°C as well as at atmospheric pressure or at a pressure above atmospheric and separating by distillation the 1,2-dichloroethane from the chlorination mixture, which comprises using, as the catalyst, anhydrous sodium tetrachloroferrate (1-) (NaFeCl$_4$), potassium tetrachloroferrate (1-) (KFeCl$_4$) or magnesium-bis-[tetrachloroferrate (1-)] (Mg[FeCl$_4$]$_2$) or using such substances in stoichiometric quantities which in the reaction mixture form these above mentioned tetrachloroferrates (1-) and carrying out the chlorination in the presence of oxygen or air the concentration of the catalyst being about 0.005 up to 0.5% by weight, calculated as ferric chloride and referred to the quantity of the solvent.

**Revendications**

1. Procédé de préparation de 1,2-dichloroéthane dans le 1,2-dichloroéthane comme solvant par réaction de l'éthylène et du chlore en présence d'un halogénure métallique qui a les propriétés d'un acide de Lewis et d'un halogénure d'un métal du groupe I A et/ou du groupe II A de la "classification périodique des éléments" comme catalyseur à une température d'environ 20 à 200°C et sous pression normale ou élevée et séparation du 1,2-dichloroéthane du mélange de chloration par distillation, caractérisé en ce que l'on utilise comme catalyseur le tétrachloroferrate (1-) de sodium (NaFeCl$_4$), le tétrachloroferrate (1-) de potassium (KFeCl$_4$) ou le bis-[tétrachloroferrate (1-)] de magnésium (Mg[FeCl$_4$]$_2$) anhydre ou des substances en quantités stoechiométriques qui forment ces tétrachloroferrates (1-) cités dans le mélange de réaction et on met en oeuvre la chloration en présence d'oxygène ou d'air, la concentration du catalyseur étant d'environ 0,005-0,5% en poids, calculée en chlorure de fer (III) et rapportée à la quantité du solvant.